# EUROPEAN PATENT SPECIFICATION

(11) **EP 0 379 516 B1**
(45) Date of publication and mention of the grant of the patent: **06.10.1993**
(21) Application number: 88908090.9
(22) Date of filing: 10.08.1988
(51) Int. Cl.: A61K 39/42

(54) **HYPER-IMMUNE GLOBULIN AGAINST HIV**
HYPERIMMUN GLOBULIN GEGEN HIV
GLOBULINE HYPER-IMMUNE CONTRE LE HIV

(30) Priority: 11.08.1987 US 84426
(43) Date of publication of application: 01.08.1990
(73) Proprietor: REGENTS OF THE UNIVERSITY OF MINNESOTA, Minneapolis, Minnesota 55455 (US); BioMune Corporation, Miami, Florida 33169 (US)
(72) Inventor: CUMMINS, Laurence, M., Libertyville, IL 60048 (US); ALLAIN, Jean-Pierre, F-75001 Paris (FR); FALK, Lawrence, A., Jr., Waukegan, IL 60085 (US); CONDIE, Richard, M., Minneapolis, MN 55405 (US); BALFOUR, Henry, H., Jr., Minneapolis, MN 55427 (US); RHAME, Frank, S., Minneapolis, MN 55414 (US)
(74) Representative: Plougmann, Ole
(86) International application number: US8802768
(87) International publication number: WO8901339

(56) References cited:
- EP-A- 0 193 284
- EP-A- 0 229 546
- Nature, vol. 316, no. 6023, July 1985, (London, GB), M. Robert-Guroff et al.: "HTLV-III-neutralizing antibodies in patients with AIDS and AIDS-related complex", pp. 72-74
- The Journal of Immunology, vol. 138, no. 4, 15 Febr. 1987, The American Association of Immunologists, (US), A.H. Rook et al.: "Sera from HTLV-III/LAV antibody-positive individuals medite antibody-positive individuals mediate antibody-dependent cellular dytotoxicity against HTLV-III/LAV-infected T cells", pp. 1064-1067

## Description

### Background of the Invention

Acquired immune deficiency syndrome, or AIDS, has come to be recognized as a public health emergency. As of December 1986, more than 27,700 Americans had been stricken by it, about half are now dead, and no one has yet recovered from the disease. Between 1 million and 1.5 million Americans have probably been infected with human immunodeficiency virus (HIV), the cause of AIDS. At least 20-50% of those infected will develop AIDS. The U.S. Public Health Service predicts that by the end of 1991, more than 179,000 persons will have succumbed to the disease. There is presently no effective treatment and a vaccine may require at least five years to develop. See Chem. Eng. News (December 8, 1986) at pages 7-14.

The effect of infection with HIV appears to vary. AIDS itself is a severe deficiency of the immune system characterized by a broad range of what are known as opportunistic infections--those caused by microorganisms that rarely cause disease in individuals with normal immune systems--and cancers resulting from the immune deficiency. Most individuals infected by the virus remain asymptomatic for various periods of time. However, there is an increasing number of infected patients who exhibit a condition known as AIDS-related complex (ARC), which is characterized by fevers, diarrhea and swollen lymph nodes. ARC is now widely considered to inevitably develop into AIDS.

Unlike many other viral infections of humans, antibody synthesis against HIV does not herald the clearing of the infection. HIV can continue to be isolated from many seropositive persons long after their initial exposure. The lentiviruses which infect other animals are known to cause infections that persist throughout the lifetime of the infected host. Unfortunately, it appears that HIV may similarly establish a persistent lifelong infection in humans. The presence of antibodies against HIV in the serum is thus not a marker of immunity, but rather, in most instances, is an indication of ongoing infection. However, antibodies which can neutralize HIV in vitro have been found in some individuals over the course of their infection with HIV. These neutralizing antibodies have been identified using assays that involve mixing cell-free virus with serum obtained from infected donors and then evaluating the infectivity of this mixture on susceptible target cells.

For example, M. Robert-Guroff and R. C. Gallo (European Patent Application No. 193,284) investigated the HIV infection of the H9 clone of the HT cell line by monitoring the expression of HIV core protein p24 using an indirect immune fluorescence assay. By three days post-infection, about 80% of the H9 cells incubated with HIV pretreated with serum of a healthy, normal donor were infected, as indicated by p24 expression. In contrast, only 10% of H9 cells expressed p24 at day three when exposed to virus which had been pretreated with serum obtained from a patient with ARC. Neutralizing antibodies were detected in 60% of adult AIDS patients and in 80% of adults with ARC, but not in healthy heterosexual controls. However, these workers did not further characterize the various sera in terms of the binding characteristics of the antibodies, but merely speculated that the neutralizing antibodies may target the major envelope proteins of HIV, e.g., gp41 and gp120.

However, it is not yet known if the immune response of such infected individuals may protect them from the development of further immunologic damage. While some changes in the pattern of serologic reactivity with specific HIV protein components during the progression from infection to disease, so far no specific patterns have been found to correlate with either the protection of health or development of disease.

In one study, J. Goudsmit et al., J. Infect. Diseases, 155, 558 (1987) used recombinant HIV core and envelope antigens to detect antibodies to these proteins in the serum of donors exhibiting different stages of HIV infection. They detected antibodies to HIV envelope antigen in all sera tested, while five of the six patients with AIDS had no detectable antibody to core antigen. However, 11 of the 13 other subjects did have detectable antibodies to this protein. HIV antigen was also assayed using a solid-phase immunoassay, and found to be present in all seven serum samples in which antibody to core was absent. The antigen was not detected in any of the 12 serum samples that contained antibody to core. Although Goudsmit et al. speculated that the absence of antibody to core HIV protein and the presence of HIV antigens in asymptomatic individuals may be predictive of future clinical disease, they did not assay any of the serum samples for HIV-neutralizing activity.

One aspect of the immune response critical to vaccine development efforts is the possible existence of a specific antibody response to HIV that could prevent, limit, or eliminate infection by the virus. Such antibodies would function as neutralizing antibodies in vivo, and an important goal for candidate vaccine immunogens is to elicit these types of antibodies.

Therefore, a need exists for methods to identify and obtain antibodies which are HIV-specific to the extent that they are efficacious for the treatment and/or prevention of AIDS.

### Brief Description of the Invention

It is generally believed that the failure to isolate HIV from all infected persons is due to technical limitations in the present T lymphocyte culture methods and to the depletion of target cells in advanced stages of the disease. However, we have discovered that an asymptomatic subset of infected persons exists who have developed antibodies in response to HIV which are effective to neutralize HIV in vivo to the extent that HIV cannot be cultured from the serum of these persons and HIV marker antigens cannot be detected in the serum by any available tissue culture and immunoassay methods. These individuals can be considered to be free of detectable replicating virus, insofar as this condition can be determined by methods available to those of skill in the art. Therefore, the present invention is based upon the identification of these individuals and the isolation of a blood fraction containing these antibodies (hereinafter referred to as HIV-hyper-immune globulin or "HIVIG") and the use of HIVIG to neutralize HIV infectivity in vitro and in vitro

Therefore, the present invention provides an HIV-specific immunoglobulin which is produced by a process comprising:
(a) identifying a human donor who is clinically healthy and whose plasma:
   (i) exhibits an anti-HIV p24 antibody titer of at least about 128;
   (ii) is HIV p24 negative by enzyme immunoassay;
   (iii) is HIV-culture negative; and
(b) isolating a sample of immunoglobulin from the blood of said donor having an HIV neutralizing titer of at least about 8000 at a concentration of about 5% by weight of immunoglobulin.

Asymptomatic individuals exposed to HIV have been identified who exhibit detectable antibodies to the HIV p24 core protein while not exhibiting detectable HIV antigen. See J. Goudsmit et al., cited hereinabove. However, it has not been appreciated that certain of these individuals can provide a source of immune globulin which is extremely high in HIV neutralizing titer. The HIV neutralizing titer is at least about 8000 and preferably is as high as 15,000-20,000, most preferably, it is greater than 50,000, as measured in a 5 wt-% solution in physiological saline by the methodology described hereinbelow.

Both the blood plasma of the donor individual and the anti-HIV immune globulin (IgG) derived from the donor exhibit a high titer of antibodies directed against HIV core p24 protein. Preferably, anti-p24 titer of the donor's plasma is at least about 128, preferably, it is about 256, most preferably about 512. The anti-p24 titer of a 5 w,t-% solution of the IgG derived from the donor's blood is at least about 10,000, preferably about 15,000, and most preferably, about 30,000. The antibody titer of the IgG composition with respect to the viral envelope proteins (gp41 and gp120) is not critical to the effectiveness of the present IgG, since the presence and titer of antibodies to these HIV proteins is exhibited by AIDS patients until they die.

The antibody titers of p24 and gp41 are determined by the competitive immunoassay developed by G. J. Dawson et al. [J. P. Allain et al., Lancet, 1233-36, (1986)] and commercialized by Abbott Laboratories, Inc., North Chicago, IL, as the "Abbott HTLV III Confirmatory EIA". (See Technical Bulletin: "Human T-Lymphotropic Virus III Antigen (Env/Core) (Recombinant E. Coli)-EIA", (93-3704/R1 February 1986), the disclosure of which is incorporated by reference herein.

The assay for HIV p24 antigen is carried out in accord with the methodology disclosed by D. A. Paul et al., J. Cell. Biochem., 10, (Suppl. A), 224 (Abstract D130) (1986) and commercialized by Abbott Laboratories, Inc., as "Abbott HTLV III Antigen EIA" as described in the technical bulletin "Antibody to Human T-Lymphotropic Virus Type III (Human)" (83-2100/R2, November 1986), the disclosure of which is incorporated by reference herein. The term "blood culture" as used with reference to the detection of HIV in donor blood is carried out as described by R. C. Gallo et al., Science, 224, 500-502 (1984), the disclosure of which is incorporated by reference herein. The term "clinically healthy" as used herein refers to the absence of symptoms of ARC or AIDS, in accord with the criteria established by the CDC (Centers for Disease Control, Atlanta, GA) (AIDS) or the working definition developed by the National Institutes of Health AIDS Working Group in collaboration with the CDC. (See Ebbesen et al., AIDS: a basic guide for clinicians. Copenhagen: Munksgaard, (1984) at page 234.) (ARC) Preferably, the T₄ lymphocyte count of the donor will be at least about 400 cells/ml of blood, most preferably about 600.

The ability to neutralize HIV infectivity in vitro with HIVIG which contains a high titer of antibodies to the HIV core viral protein, p24, can provide the basis for assays of the effectiveness of some prototype HIV vaccines, and will also be useful in basic investigations into the mechanisms of HIV infection, e.g., with respect to the effect of mutations on the products of viral gene expression.

The present invention is also directed to a method for raising plasma antibody titers to HIV p24 by administering a pharmaceutical unit dosage form of the present HIVIG to a patient who has been exposed to HIV. Therefore, it is believed that the HIVIG of the present invention will have therapeutic potential, either as a prophylactic agent to prolong the life expectancy of infected and/or diseased patients by retarding the clinical progression of the disease, or possibly, as a curative agent which can act to eliminate the virulence of the virus. HIVIG might also represent a valuable adjunct to the antiviral agents, AZT (azidothymidine), DDC (dideoxycytidine), or other potentially effective drugs. As discussed hereinabove, there has heretofore been no reason to believe that antibodies capable of neutralizing HIV in vitro would be effective to inhibit the progression of AIDS. However, the absence of the clinical symptoms of ARC or AIDS in the HIVIG donors, coupled with the absence of both viral infectivity and p24 antigen in their serum, provides strong additional empirical evidence that the HIVIG will be effective in vivo.

It is to be appreciated that the present source of HIVIG is necessarily donors who have been previously exposed to HIV. However, the development of an essentially similar antibody profile in healthy subjects following their innoculation with vaccines derived from immunogenic viral proteins or the fragments thereof, preferably derived via recombinant viral DNA, is expected to provide a more controlled source of HIVIG.

### Detailed Description of the Invention

The IgG of the present invention is isolated from pooled plasma obtained by plasmapheresis from selected HIV-exposed donors who have no significant active viral replication. The donors were selected from a pool of asymptomatic sero-converted subjects who subsequently exhibited the anti-HIV antibody profiles described hereinbelow, while testing negative for HIV antigen. The plasma of the donors was also unable to infect HIV-susceptible cells.

Any contaminating viruses in the plasma pool can be inactivated by alcohol fractionation. Morbidity and Mortality Weekly Report, 35, 231 (April 11, 1986). IgG was derived from the plasma by centrifugal fractionation in the presence of cold ethanol (-10°C-0°C) at neutral pHs (6.8-7.3). [See E. J. Cohn, U.S. Patent Nos. 2,390,074 and 2,770,616]. The desired IgG can also be obtained by the controlled protein fractionation method described by G. U. Bethel and R. M. Condie, in U.S. Patent application No. 36,031, filed May 4, 1979. The resultant IgG-containing paste was suspended in excess distilled water and lyophilized at less than 0°C to remove residual ethanol.

The IgG product preferably is diluted with a pharmaceutically-acceptable liquid carrier, such as an aqueous IV fluid, prior to being assayed for bioactivity or administered as a unit dosage form in vivo. See Remington's Pharmaceutical Sciences, A. Osol, ed., Mack Pub. Co., Easton, PA, (16th ed. 1980) at pages 1488-1496, the disclosure of which is incorporated by reference herein. The resultant solution is sterilized, e.g., by filtration. Preservatives commonly employed with IgG preparations, such as maltose, glycine or thimerosal, may be added in pharmaceutically acceptable amounts.

The resulting solutions are preferably administered parenterally, e.g., by intravenous infusion or injection. The amount of IgG administered will vary widely, and will depend on the physique and physical condition of the HIV-infected patient. Such factors are necessarily empirical, and can be determined by the clinician, employing the ARC or AIDS staging criteria described hereinabove. In some clinical situations, it may be necessary to administer a plurality of doses of the IgG composition, in order to neutralize the infectivity of viral particles as they are released from infected target cells.

The invention will be described by reference to the following detailed example.

### EXAMPLE - ISOLATION OF HIGH TITER ANTI-P24 IMMUNE GLOBULIN (HIVIG)

### A. Immunoassay Methodology

1. Detection of HIV antigen. Serum samples were assayed for HIV antigen in a solid-phase immunoassay (Abbott Laboratories, North Chicago, IL) as generally described by J. Goudsmit et al., Lancet, 177 (1986). Two hundred microliters of sample were incubated overnight at room temperature (about 22°C) with a bead coated with human antibody to HIV. Beads were washed with distilled water; rabbit IgG antibody to HIV was added and incubated for 4 hr at 45°C. Beads were washed as before and then incubated for 2 hr at 45°C with horseradish peroxidase-conjugated goat antibody to rabbit IgG. After a final wash, the beads were transferred to tubes and o-phenylenediamine was added. After 30 min at room temperature in the dark, 1 ml of H₂SO₄ was added to each tube. THe A₄₉₂ was read by using the Quantum Dual Wavelength Spectrophotometer (Abbott Laboratories). A sample was considered positive if its OD was ≧0.050 plus the mean of five duplicates of normal human plasma. The assay is most sensitive for the core antigen of HIV, p24, and detects antigen in culture supernatant of 10³ cells/ml of HIV--infected HT-9 cells (uninfected HT-9 cell supernatant is negative). The specificity of the assay is determined in part by the rabbit antibody which, when used on Western Blots of purified HIV lysate or in RIPA procedures, detects p55/24 (core) strongly and gp120/41 (envelope) only faintly. Purified, recombinant core antigen is detectable at 50 pg/ml when spiked into serum or plasma, whereas purified, recombinant envelope antigen is detectable at about 500 ng/ml. Quantitation of HIV antigen was done by comparing the ODs of the samples with the ODs of known quantities of purified HIV lysate. Using this antigen as reference, the detection limit of this assay is about 0.05 ng/ml.
2. Detection of antibodies to HIV core and envelope proteins. Antibodies were measured by using commercially available competitive enzyme immunoassays (Abbott Laboratories, North Chicago. IL) as generally described by J. P. Allain et al., Lancet, 1233-36 (1986). Briefly, in the first system, beads coated with recombinant HIV core antigen containing the entire p24gag gene product, as well as portions of the p15- and p18-gag gene products) were incubated with 50 µl of serial twofold dilutions of the serum samples (diluted in normal human plasma) and with 200 µl of horseradish peroxidase-conjugated human antibody to HIV core antigen for 16-22 hr at room temperature. Beads were washed with distilled water, and color was developed as described above for the antigen test. Because this is a competitive enzyme immunoassay, intensity of the color formed was inversely related to the amount of antibody to the HIV core antigen in the sample. The cutoff point was determined as the sum of the negative control mean (n = 3) and the positive control mean (n = 2), divided by two. Antibody titer was considered as the highest dilution at which the A₄₉₂ of the sample was less than the cutoff. The same procedure applied to the assay for antibody to envelope antigen; however, beads were coated with recombinant HIV envelope antigen (containing the entire p41env gene product, as well as 45 amino acids from the gp120env gene product), and horseradish peroxidase-conjugated human antibody to HIV envelope was used instead of antibody to core. Labeled antibodies in the assays for antibody to core and antibody to envelope were prepared from the sera of patients with high titers to sore or envelope, respectively, on Western Blot.

### B. Selection of Donors

Serum samples were obtained from 25 individuals who had previously tested positive for HIV antibody. The level of anti-p24 and anti-gp41 antibody were measured by the Abbott LaboratorieS competitive enzyme immunoassay described in section A(2), here-inabove. The gp120 antibody was detected by Western Blot but not quantitated. See J. P. Allain et al., supra. HIV antigen was tested for by both culture and by the immunoassay described in section A(1) above. From the 25 individuals, 11 were asymptomatic, and plasma antigen negative while exhibiting with relatively high HIV antibody titers. Three of these donors were selected as plasma donors who were all blood HIV culture negative and HIV antigen negative. They were clinically healthy with T₄ lymphocyte counts of ≧400/ml. The donors met the requirements of the Code of Federal Regulations [21 C.F.R. §§640.60-640.63 (April 1, 1986)] for source plasma, with the exception that they were HIV antibody positive. The donors were plasmapheresed according to CFR requirements and plasma flash frozen and stored at less than 0°C until fractionated [21 C.F.R. §640.65 (April 1, 1986)]. The criteria for selection of these three donors were: Donor JD exhibited a low titer of anti-gp120, non-detectable antibody to p24 and moderate titer to gp41; Donor MG exhibited a high titer to gp41 and gp120 and a non-detectable titer of anti-p24; and Donor DE exhibited a high titer to p24 and gp120, and a low titer to gp41. These HIV antibody profiles are summarized on Table 1, below.

**TABLE 1**

| HIV ANTIBODY PROFILE ON DONOR PLASMA | | | | | |
|---|---|---|---|---|---|
| Donor | ElA ANTI-p24 | ElA ANTI-gp41 | Western Blot¹ | | |
| | | | gp41 | p24 | gp120 |
| JD | Negative | 4096 | S | M | N |
| MG | Negative | 8684 | S | M | S |
| DE | 18842 | 2048 | S | S | S |

| | | | | | |
|---|---|---|---|---|---|
| ¹ Visually quantitated S-Strong, M-Mild, N-Non-detectable. | | | | | |

Two units of plasma from each donor were pooled to provide a starting pool of approximately 1200 ml. The three separate pools were fractionated by the procedure reported by Cohn et al., J. Clin. Invest., 23, 417 (1944), the disclosure of which is incorporated by reference herein. The residual ethanol was removed from the resultant "Fraction II" IgG paste by lyophilization and a 5.0% wt/wt IgG solution was made from the powders by dissolving them in 0.9% saline. The solutions we filtered through 0.2 micron sterile filters and stored under sterile conditions prior to testing their neutralizing activity. No preservatives or stabilizing agents were added. The control lot (5% IgG) was prepared from IgG isolated from donors who tested non-reactive for HIV.

### C. Determination of Neutralization Activity

The 5% IgG solutions were heat-inactivated for 30 min at 56°C, filtered through a 0.45 micron filter and serial dilutions were made in tissue culture media. (RPMI - 1640 with 10% fetal calf serum (FCS) supplemented with penicillin and streptomycin). One half ml of the diluted sample was mixed with 0.5 ml of HIV (100 infectious units/ml) and incubated at 37°C with 5% CO₂ for two hours. One half ml is discarded and 4.0 ml of H9 cells (5 × 10⁵ cells/ml) are added and the cultures incubated at 37°C with 5% CO₂. After 5 to 7 days, 0.45 ml of the culture was removed, cells lysed by adding 0.05 ml of 5% Triton X-100. Cell lysates were monitored for HIV antigen at 5- to 7-day intervals for a period of 3 weeks. HIV antigen concentrations were determined by a solid phase immunoassay (Abbott Laboratories, North Chicago, IL). Inhibition of HIV replication (neutralization) is indicated by optical density values less than the assay control (normal human serum negative for HIV antibody).
The HIV neutralization titer is reported as the highest dilution that gives an optical density value reading less than the assay control. Neutralization titers for the 5% IgG solutions are presented in Table 2, below.

**TABLE 2**

| IgG Solution | ANTI-p24 Titer | ANTI-gp41 Titer | ANTI-gp120 Titer | Neutralization Titer |
|---|---|---|---|---|
| Control IgG | Negative | Negative | <1 | <2 |
| JD-1 | Negative | 8192 | <20,000 | <2 |
| MG-1 | Negative | 16384 | >20,000 | <2 |
| DE-2 | 16384 | 1024 | >20,000 | ≧65,000 |

### D. Discussion

The data summarized on Table 2 indicate that only the IgG solution derived from donor DE-2 contained an appreciable quantity of anti-p24 antibody, and that only this solution could neutralize HIV in vitro. It is also apparent that solutions containing high anti-gp41 and anti-gp120 antibodies, without anti-p24, had little or no neutralizing activity. This data along with serological profile data that show no antigenemia in the presence of anti-p24 antibodies suggest the anti-core antibody and not the anti-envelope antibody is an excellent serological marker to identify donors which can provide neutralizing antibody. It is believed that HIVIG derived from asymptomatic donors which is high titer in anti-p24 antibodies may be useful in vivo for the inhibition of the progression of HIV infection or for the treatment of ARC or AIDS patients.

The invention has been described with reference to various specific and preferred embodiments and techniques. However, it should be understood that many variations and modifications may be made while remaining within the spirit and scope of the invention.

## Claims

1. An immunoglobulin (IgG) which is produced by a process comprising:
(a) identifying a human donor who is infected with HIV, clinically healthy and whose plasma:
(i) exhibits an anti-HIV p24 antibody titer of at least about 128;
(ii) is HIV p24 negative by enzyme immuno-assay;
(iii) is HIV-culture negative; and
(iv) has a T₄ lymphocyte count of at least about 400/ml of blood; and
(b) isolating a sample of immunoglobulin from the blood of said donor having an HIV neutralizing titer effective to neutralize the infectivity of HIV.

2. A method for the in vitro neutralization of HIV, comprising:
treating an amount of HIV with an amount of the IgG of claim 1 in a pharmaceutically acceptable carrier, which is effective to neutralize the infectivity of the HIV.

3. A composition for neutralizing the infectivity of HIV, comprising:
an amount of immunoglobulin (IgG) effective to neutralize the infectivity of HIV, said IgG being obtained from blood plasma exhibiting an anti-HIV p24 antibody titer of at least 128, and a T₄ lymphocyte count of at least 400/ml of blood.

4. The composition of claim 3 which is adapted for parenteral administration.

5. The composition of claim 3 which is adapted for intravenous injection or infusion.

6. An immunoglobulin (IgG) effective to neutralize the infectivity of HIV, said IgG being obtained from blood plasma exhibiting an anti-HIV p24 antibody titer of at least 128, and a T₄ lymphocyte count of at least 400/ml of blood for use as a therapeutic agent.

7. Use of an immunoglobulin (IgG) effective to neutralize the infectivity of HIV, said IgG being obtained from blood plasma exhibiting an anti-HIV p24 antibody titer of at least 128, and a T₄ lymphocyte count of at least 400/ml of blood for the preparation of a pharmaceutical composition for the prophylaxis or treatment of HIV infection.

## Patentansprüche

1. Immunoglobulin (IgG), welches nach einem Verfahren hergestellt ist, welches umfaßt:
(a) Identifizierung eines menschlichen Spenders, der mit HIV infiziert ist, klinisch gesund ist und dessen Plasma:
(i) einen Anti-HIV p24-Antikörpertiter von wenigstens etwa 128 zeigt;
(ii) HIV p24 negativ beim Enzymimmunoassay ist;
(iii) HIV-Kultur-negativ ist; und
(iv) eine T₄-Lymphocytenzahl von wenigstens etwa 400/ml Blut aufweist; und
(b) Isolierung einer Probe von Immunoglobulin aus dem Blut dieses Spenders, das einen HIV-Neutralisationstiter aufweist, der zur Neutralisation der Infektiosität von HIV effektiv ist.

2. Verfahren zur in vitro Neutralisation von HIV, umfassend:
Behandlung einer Menge von HIV mit einer Menge des IgG von Anspruch 1 in einem pharmazeutisch annehmbaren Träger, die zur Neutralisation der Infektiosität des HIV effektiv ist.

3. Zusammensetzung zur Neutralisation der Infektiositat von HIV, umfassend:
eine Menge von Immunoglobulin (IgG), die zur Neutralisation der Infektiosität von HIV effektiv ist, wobei dieses IgG aus Blutplasma erhalten wurde, das einen Anti-HIV p24-Anti-körpertiter von wenigstens 128 und eine T₄-Lymphocytenzahl von wenigstens 400/ml Blut aufweist.

4. Zusammensetzung nach Anspruch 3, welche für parenterale Applikation angepaßt ist.

5. Zusammensetzung nach Anspruch 3, welche für intravenöse Applikation angepaßt ist.

6. Zur Neutralisation der Infektiosität von HIV effektives Immunoglobulin (IgG), wobei dieses IgG aus Blutplasma erhalten wurde, das einen Anti-HIV p24-Antikörpertiter von Wenigstens 128 und eine T₄-Lymphocytenzahl von Wenigstens 400/ml Blut aufweist, zur Verwendung als therapeutisches Mittel.

7. Verwendung eines zur Neutralisation der Infektiosität von HIV effektiven Immunoglobulins (IgG), wobei dieses IgG aus Blutplasma erhalten wurde, das einen Anti-HIV p24-Anti-körpertiter von wenigstens 128 und eine T₄-Lymphocytenzahl von wenigstens 400/ml Blut aufweist, zur Herstellung einer Pharmazeutischen Zusammensetzung für die Prophylaxe oder Behandlung von HIV-Infektion.

## Revendications

1. Une immunoglobuline (IgG) produite par un procédé comprenant :
(a) l'identification d'un donneur humain infecté par le HIV cliniquement sain et dont le plasma :
(i) présente un titre en anticorps anti-p24 de HIV d'au moins 128 ;
(ii) donne une réaction négative avec le p24 de HIV en test imunoenzymatique ;
(iii) donne une réaction négative en culture avec HIV ;
(iv) présente un taux de lymphocytes T4 d'au moins 400 par ml de sang ; et
(b) l'isolement d'un échantillon d'immunoglobuline à partir du sang dudit donneur ayant un titre de neutralisation de HIV efficace pour neutraliser le caractère infectieux du HIV.

2. Méthode pour la neutralisation in vitro de HIV comprenant le traitement d'une quantité de HIV, avec une quantité d'IgG selon la revendication 1 dans un véhicule pharmaceutiquement acceptable, laquelle quantité étant efficace pour neutraliser le caractère infectieux du HIV.

3. Composition pour neutraliser le caractère infectieux de HIV comprenant :
(a) une quantité d'immunoglobuline (IgG) efficace pour neutraliser le caractère infectieux de HIV, ladite IgG était obtenue à partir de plasma sanguin présentant un titre en anticorps anti-p24 de HIV d'au moins 128 et un taux de lymphocytes T4 d'au moins 400/ml de sang.

4. Composition selon la revendication 3, adaptée pour l'administration parentérale.

5. Composition selon la revendication 3, adaptée pour l'injection ou l'infusion intraveineuse.

6. Une immunoglobuline (IgG) efficace pour neutraliser le caractère infectieux de HIV, ladite IgG présentant un titre en anticorps anti-p24 de HIV d'au moins 128 et un taux de lymphocytes T4 d'au moins 400/ml de sang, pour l'utilisation en tant qu'agent thérapeutique.

7. Utilisation d'une immunoglobuline (IgG) efficace pour neutraliser le caractère infectieux de HIV, ladite IgG présentant un titre en anticorps anti-p24 de HIV d'au moins 128 et un taux de lymphocytes T4 d'au moins 400/ml de sang, pour la préparation d'une composition pharmaceutique pour la prophylaxie ou le traitement de l'infection par HIV.
